# EUROPEAN PATENT SPECIFICATION

(11) **EP 3 100 052 B1**
(45) Date of publication and mention of the grant of the patent: **10.03.2021**
(21) Application number: 15743166.9
(22) Date of filing: 29.01.2015
(51) Int. Cl.: G01N 33/574, C12Q 1/6886

(54) **CIRCULATING TUMOR CELL DIAGNOSTICS FOR BIOMARKERS PREDICTIVE OF RESISTANCE TO ANDROGEN RECEPTOR (AR) TARGETED THERAPIES**
DIAGNOSE ZIRKULIERENDER TUMORZELLEN FÜR BIOMARKER ZUR VORHERSAGE DER RESISTENZ GEGEN AUF DEN ANDROGENEN REZEPTOR ABZIELENDE THERAPIEN
DIAGNOSTIC BASÉ SUR LES CELLULES TUMORALES CIRCULANTES À LA RECHERCHE DE BIOMARQUEURS PRÉDICTIFS D'UNE RÉSISTANCE AUX THÉRAPIES CIBLANT LE RÉCEPTEUR DES ANDROGÈNES (AR)

(30) Priority: 30.01.2014 US 201461933774 P
(43) Date of publication of application: 07.12.2016
(73) Proprietor: Epic Sciences, Inc., San Diego, CA 92121 (US)
(72) Inventor: DITTAMORE, Ryan, San Diego, CA 92121 (US)
(74) Representative: Jones Day
(86) International application number: PCT/US2015/013538
(87) International publication number: WO 2015/116828

(56) References cited:
- WO-A1-2014/008155
- WO-A1-2015/048740
- US-A1- 2012 276 555
- M. S. DARSHAN ET AL: "Taxane-Induced Blockade to Nuclear Accumulation of the Androgen Receptor Predicts Clinical Responses in Metastatic Prostate Cancer", CANCER RESEARCH, vol. 71, no. 18, 28 July 2011 (2011-07-28) , pages 6019-6029, XP055386476, us ISSN: 0008-5472, DOI: 10.1158/0008-5472.CAN-11-1417
- NAGRATH SUNITHA ET AL: "Isolation of rare circulating tumour cells in cancer patients by microchip technology", NATURE, MACMILLAN JOURNALS LTD., ETC, vol. 450, no. 7173, 20 December 2007 (2007-12-20), pages 1235-1239, 1, XP002576863, ISSN: 0028-0836, DOI: 10.1038/NATURE06385
- XIAOTUN ZHANG ET AL: "Androgen Receptor Variants Occur Frequently in Castration Resistant Prostate Cancer Metastases", PLOS ONE, vol. 6, no. 11, 17 November 2011 (2011-11-17), page e27970, XP055266797, DOI: 10.1371/journal.pone.0027970
- EDWIN E REYES ET AL: "Quantitative characterization of androgen receptor protein expression and cellular localization in circulating tumor cells from patients with metastatic castration-resistant prostate cancer", JOURNAL OF TRANSLATIONAL MEDICINE, BIOMED CENTRAL, LONDON, GB, vol. 12, no. 1, 26 November 2014 (2014-11-26), page 313, XP021206180, ISSN: 1479-5876, DOI: 10.1186/S12967-014-0313-Z
- HOWARD I. SCHER ET AL: "Association of AR-V7 on Circulating Tumor Cells as a Treatment-Specific Biomarker With Outcomes and Survival in Castration-Resistant Prostate Cancer", JAMA ONCOLOGY, vol. 2, no. 11, 1 November 2016 (2016-11-01), page 1441, XP055387796, ISSN: 2374-2437, DOI: 10.1001/jamaoncol.2016.1828
- MIYAMOTO ET AL.: 'Androgen Receptor Signaling in Circulating Tumor Cells as a Marker of Hormonally Responsive Prostate Cancer' CANCER DISCOVERY vol. 2, no. 11, November 2012, pages 995 - 1003, XP055211802
- MARRINUCCI ET AL.: 'Fluid biopsy in patients with metastatic prostate, pancreatic and breast cancers' PHYS. BIOL vol. 9, no. 1, 03 February 2012, pages 1 - 9, XP020218196
- LEVERSHA ET AL.: 'Fluorescence in situ Hybridization Analysis of Circulating Tumor Cells in Metastatic Prostate Cancer''.' CLIN CANCER RES vol. 15, no. 6, 15 March 2009, pages 2091 - 2097, XP055020139
- ZHANG ET AL.: 'Androgen Receptor Variants Occur Frequently in Castration Resistant Prostate Cancer Metastases' PLOS ONE vol. 6, no. 11, page E27970, XP055266797
- RICKMAN ET AL.: 'ERG Cooperates with Androgen Receptor in Regulating Trefoil Factor 3 in Prostate Cancer Disease Progression' NEOPLASIA vol. 12, no. 12, December 2010, pages 1031 - 1040, XP055357438
- NAGLE ET AL.: 'ERG Overexpression and PTEN Status Predict Capsular Penetration in Prostate Carcinoma' PROSTATE vol. 73, no. 11, August 2013, pages 1233 - 1240, XP055357439
- Anonymous: "Circulating tumor cell", Wikipedia, 13 January 2015 (2015-01-13), XP055472147, Retrieved from the Internet: URL:https://en.wikipedia.org/w/index.php?t itle=Circulating_tumor_cell&oldid=64223529 5 [retrieved on 2018-05-03]
- RACILA E ET AL: "Detection and charcterization of carcinoma cells in the blood", PROCEEDINGS OF THE NATIONAL ACADEMY OF SCIENCES, NATIONAL ACADEMY OF SCIENCES, US, vol. 95, 1 April 1998 (1998-04-01), pages 4589-4594, XP002132943, ISSN: 0027-8424, DOI: 10.1073/PNAS.95.8.4589
- PETER SIDAWAY: "Non-traditional CTCs indicate prognosis : Urological cancer", NATURE REVIEWS CLINICAL ONCOLOGY, vol. 13, no. 10, 7 September 2016 (2016-09-07), pages 592-592, XP055472116, NY, US ISSN: 1759-4774, DOI: 10.1038/nrclinonc.2016.146
- R.M. Bambury ET AL: "237PCHARACTERISTICS OF DE NOVO RESISTANCE TO ANDROGEN TARGETING THERAPEUTICS (AR TX) THROUGH CIRCULATING TUMOR CELLS (CTCS) ANALYSIS IN METASTATIC CASTRATION RESISTANT PROSTATE CANCER (MCRPC) PATIENTS", ANNALS OF ONCOLOGY., vol. 25, no. suppl_4, 1 September 2014 (2014-09-01), pages iv79-iv79, XP055450387, NL ISSN: 0923-7534, DOI: 10.1093/annonc/mdu326.70
- T. A. Yap ET AL: "Circulating Tumor Cells: A Multifunctional Biomarker", CLINICAL CANCER RESEARCH, vol. 20, no. 10, 14 May 2014 (2014-05-14), pages 2553-2568, XP055568616, US ISSN: 1078-0432, DOI: 10.1158/1078-0432.CCR-13-2664
- YIEN NING SOPHIA WONG ET AL: "Evolution of androgen receptor targeted therapy for advanced prostate cancer", NATURE REVIEWS CLINICAL ONCOLOGY, vol. 11, no. 6, 20 May 2014 (2014-05-20), pages 365-376, XP55567743, NY, US ISSN: 1759-4774, DOI: 10.1038/nrclinonc.2014.72

## Description

The invention relates generally to the field of cancer diagnostics and, more specifically to methods for predicting resistance to AR targeted therapies in a prostate cancer patient.

### BACKGROUND

Prostate cancer (PC) remains the most common non-cutaneous cancer in the US. In 2014 alone, the projected incidence of prostate cancer is 233,000 cases with deaths occurring in 29,480 men, making metastatic prostate cancer therapy truly an unmet medical need. Siegel et al., 2014. CA Cancer J Clin. 2014;64(1):9-29. Epidemiological studies from Europe show comparable data with an estimated incidence of 416700 new cases in 2012, representing 22.8% of cancer diagnoses in men. In total, 92200 prostate cancer deaths are expected, making it one of the three cancers men are most likely to die from, with a mortality rate of 9.5%

With the advent of exponential growth of novel agents tested and approved for the treatment of patients with metastatic castration-resistant prostate cancer (mCRPC) in the last 5 years alone, issues regarding the optimal sequencing or combination of these agents have arisen. Several guidelines exist that help direct clinicians as to the best sequencing approach and most would evaluate presence or lack of symptoms, performance status, as well as burden of disease to help determine the best sequencing for these agents. Mohler et al., 2014, J Natl Compr Canc Netw. 2013;11(12):1471-1479; Cookson et al., 2013, J Urol. 2013;190(2):429-438. Currently, approved treatments consist of taxane-class cytotoxic agents such as Taxotere® (docetaxel) and Jevtana® (cabazitaxel), and anti-androgen hormonal therapy drugs such as Zytiga® (abiraterone, blocks androgen production) or Xtandi® (enzalutamide, an androgen receptor (AR) inhibitor).

The challenge for clinicians is to decide the best sequence for administering these therapies to provide the greatest benefit to patients. However, therapy failure remains a significant challenge based on heterogeneous responses to therapies across patients and in light of cross-resistance from each agent. Mezynski et al., Ann Oncol. 2012;23(11):2943-2947;. Noonan et al., Ann Oncol. 2013;24(7):1802-1807; Pezaro et al., Eur Urol. 2014, 66( 3): 459-465. In addition, patients may lose the therapeutic window to gain substantial benefit from each drug that has been proven to provide overall survival gains. Hence, better methods of identifying the target populations who have the most potential to benefit from targeted therapies remain an important goal.

Circulating tumor cells (CTCs) represent a significant advance in cancer diagnosis made even more attractive by their non-invasive measurement. Cristofanilli et al., N Engl J Med 351:781-91, (2004) CTCs released from either a primary tumor or its metastatic sites hold important information about the biology of the tumor. Quantifying and characterizing CTCs, as a liquid biopsy, assists clinicians to select the course of therapy and to watch monitor how a patient's cancer evolves. CTCs can therefore be considered not only as surrogate biomarkers for metastatic disease but also as a promising key tool to track tumor changes, treatment response, cancer recurrence or patient outcome non-invasively. Historically, the extremely low levels of CTCs in the bloodstream combined with their unknown phenotype has significantly impeded their detection and limited their clinical utility. A variety of technologies are presently being developed developed for detection, isolation and characterization of CTCs in order to utilize their information.

Bambury et al. disclose that metastatic castration resistant prostate cancer patients with *de novo* resistance to androgen targeting therapy have a higher baseline frequency of CTCs (traditional CTCs, cytokeratin negative CTCs and androgen receptor expressing CTCs) than responders. Bambury et al., 2014. Annals of Oncology 25 (Suplement 4): iv58-iv84.

A need exists to develop accurate and non-invasive methods for determining the optimal sequence to administer AR targeted and taxane-based chemotherapy to maximize individual patient benefit. The present invention addresses this need by providing biomarker signatures that predict resistance to AR targeted therapies and chemotherapy based on a robust CTC detection and characterization platform that enables phenotypic characterization of CTCs. Related advantages are provided as well.

### SUMMARY OF THE INVENTION

The present disclosure provides CTC methods for prospectively identifying resistanceto AR targeted therapies and chemotherapy in a prostate cancer patient.

The disclosure provides a method of predicting resistance to androgen receptor (AR) targeted therapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to AR targeted therapy in the prostate cancer patient. In some embodiments, the resistance is *de novo* resistance.

In some embodiments, the present disclosure provides a method of predicting resistance to androgen receptor (AR) targeted therapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to AR targeted therapy in the prostate cancer patient, wherein the biomarker signature comprises CK+, AR+, nucleoli+ CTCs in a subpopulation of said CTCs. In some embodiments, the resistance is *de novo* resistance.

In further embodiments, the present disclosure provides a method of predicting resistance to androgen receptor (AR) targeted therapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to AR targeted therapy in the prostate cancer patient, wherein the biomarker signature comprises presence of AR N-terminal positive CTCs and AR C-terminal loss. In some embodiments, the resistance is de novo resistance.

In further embodiments, the present disclosure provides a method of predicting resistance to androgen receptor (AR) targeted therapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to AR targeted therapy in the prostate cancer patient, wherein the biomarker signature comprises increased heterogeneity of the CTCs compared to a reference population. In some embodiments, the resistance is de novo resistance.

The present disclosure also provides a method of predicting resistance to chemotherapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to chemotherapy in the prostate cancer patient. In some embodiments, the resistance is *de novo* resistance.

The present disclosure also provides a method of predicting resistance to taxane-based chemotherapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to taxane-based chemotherapy in the prostate cancer patient, wherein the biomarker signature comprises CK+, AR-, nucleoli+ , small size in a subpopulation of said CTCs. In some embodiments, the resistance is *de novo* resistance.

The invention provides a method of predicting resistance to androgen receptor (AR) targeted therapy in a prostate cancer patient comprising
(a) performing a direct analysis comprising immunofluorescent staining and morphological characterization of nucleated cells in a blood sample obtained from the patient to identify circulating tumor cells (CTCs), wherein the direct analysis means the detection of CTCs in the context of all surrounding nucleated cells present in the sample as opposed to after enrichment of the sample for CTCs prior to detection; and
(b) based on said direct analysis further determining the presence of a biomarker signature that is predictive of resistance to AR targeted therapy in the prostate cancer patient,
wherein the biomarker signature comprises a subpopulation of said CTCs which is cytokeratin positive (CK+), AR positive (AR+), and comprises nucleoli (nucleoli+),
and wherein said CTCs are further characterized by AR N-terminal positive staining and AR C-terminal loss.

In certain aspects of the invention, the immunofluorescent staining of nucleated cells comprises pan cytokeratin (CK), cluster of differentiation (CD) 45, diamidino-2-phenylindole (DAPI) and AR. In additional aspects, the AR immunofluorescent staining comprises N- terminal and C-terminal AR nuclear staining.

In some aspects of the invention, the prediction of resistance to AR targeted therapy informs a subsequent treatment decision. In particular embodiments, the prostate cancer is metastatic castration resistant prostate cancer (mCRPC).

Other features and advantages of the invention will be apparent from the detailed description, and from the claims.

### BRIEF DESCRIPTION OF THE DRAWINGS

Figure 1 shows a graph depicting patterns of PSA changes after AR signaling directed therapies.

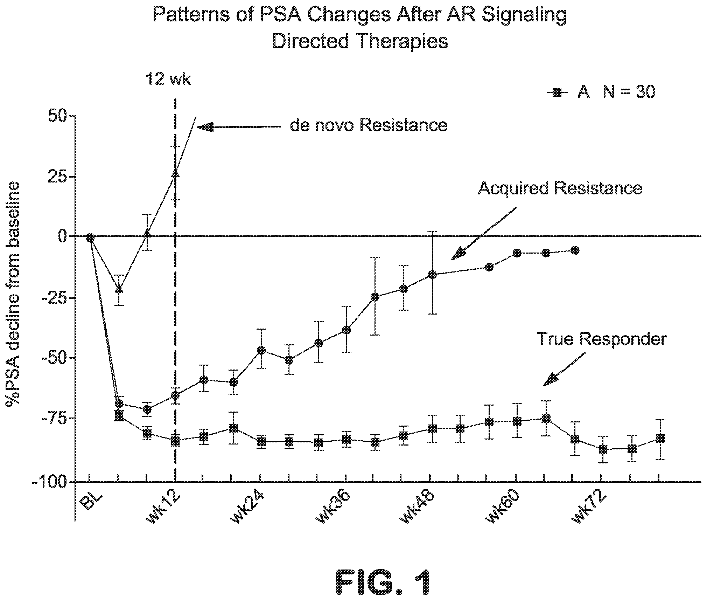

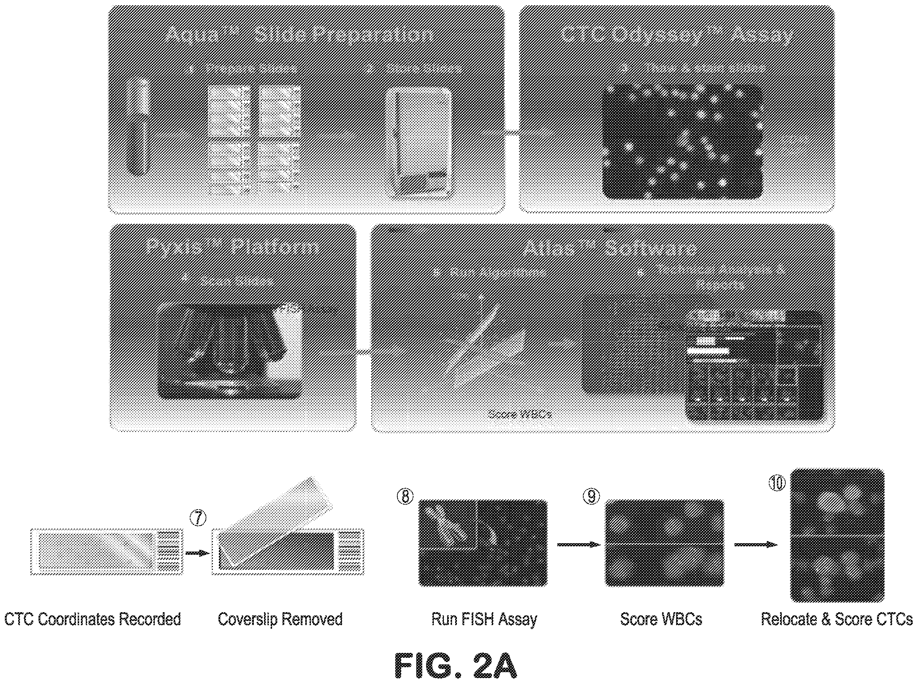

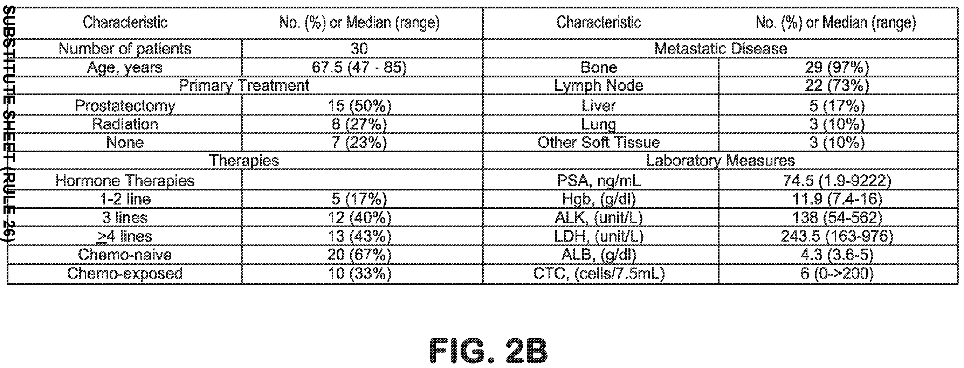

Figures 2A and 2B show methods used in performing the embodiments exemplified herein and patient demographics of the study group. Figure 2A shows a schematic of a representative CTC collection and detection process: (1) nucleated cells from blood sample placed onto slides; (2) slides stored in -80°C biorepository; (3) slides stained with CK, CD45, DAPI and AR; (4) slides scanned; (5) multi-parametric digital pathology algorithms run; (6) software and human reader confirmation of CTCs and quantitation of biomarker expression; (7) for FISH, coordinates are recorded and coverslip removed; (8) FISH assay is run; (9) regional WBCs are scored to assess normal; and (10) CTCs relocated and scored. Figure 1B shows information on the study population. Demographic and clinical characteristics of patients at the time of inclusion in the study are shown in the left and right panels. Thirty progressive mCRPC patients (pts) were included in the study.
Figure 3 shows Epic vs. CellSearch® Frequency with EPIC CTCs/mL extrapolated to CTCs/7.5 mL vs. CellSearch CTCs/7.5 mL. Matched blood samples were processed utilizing CellSearch® and Epic Sciences CTC platform. CellSearch® enumeration limited to 200 CTCs. Epic CTCs were measured from 1mL and extrapolated to 7.5 mL of blood.
Figure 4 shows immunofluorescence images of CTC subpopulations detected on the Epic platform.

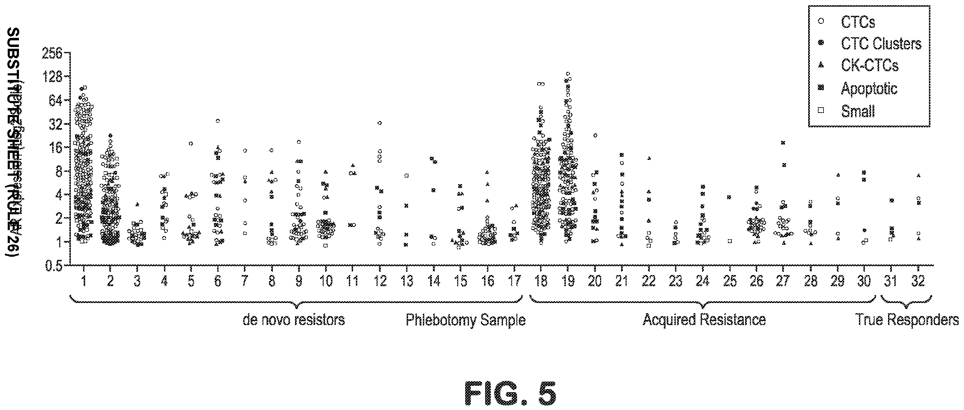

Figure 5 shows the heterogeneity of AR expression of observed CTCs and CTC subpopulations.

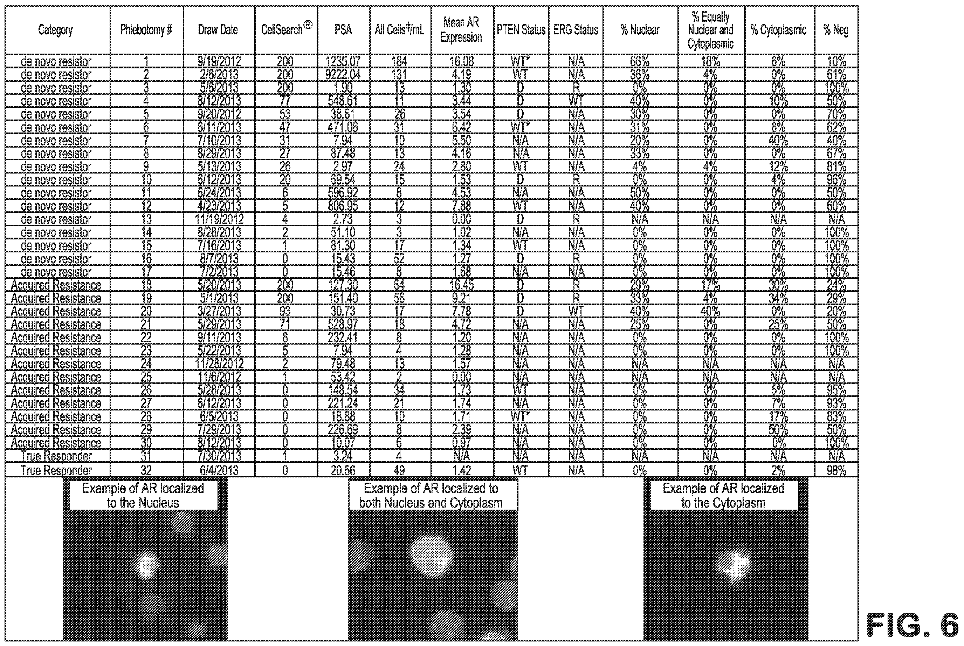

Figure 6 shows CTC characterization and AR localization heterogeneity. All Cells include traditional CTCs, Apoptotic CTCs, CK- CTCs and Small CTCs.

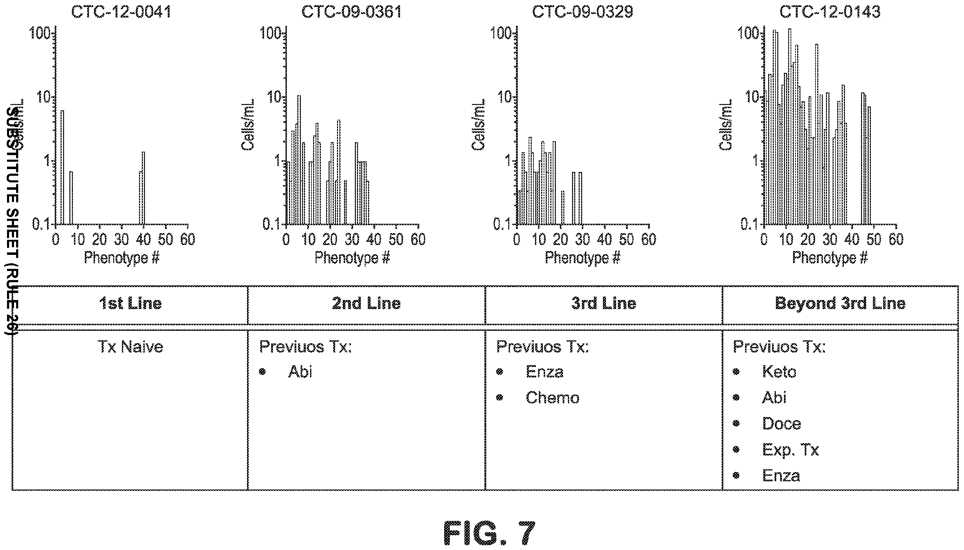

Figure 7 shows CTC heterogeneity observed in samples of patients after various lines of therapy. To determine heterogeneity, each cell is bucketed into 1 of 70 categories. Samples with more non-zero types, and therefore more non-zero bars on the graph, are considered more heterogeneous than samples with a few non-zero populations. The lower panel shows the therapies the patient from who the sample was obtained had undergone at the time the sample was taken.

### DETAILED DESCRIPTION

The present disclosure is based, in part, on the identification of a CTC biomarker signature that enables prospective prediction of resistance to AR targeted therapies in a patient afflicted with mCRPC. The present disclosure is further based, in part, on the identification of a CTC biomarker signature that enables prospective prediction of resistance to chemotherapy in a patient afflicted with mCRPC. The optimal sequence to administer AR targeted and taxane-based chemotherapy to maximize individual patient benefit is an unmet medical need.

The present disclosure provides a method of predicting resistance to androgen receptor (AR) targeted therapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to AR targeted therapy in the prostate cancer patient. In some embodiments, the resistance is *de novo* resistance.

In some embodiments, the present disclosure provides a method of predicting resistance to androgen receptor (AR) targeted therapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to AR targeted therapy in the prostate cancer patient, wherein the biomarker signature comprises CK+, AR+, nucleoli+ CTCs in a subpopulation of said CTCs. In some embodiments, the resistance is *de novo* resistance.

In further embodiments, the present disclosure provides a method of predicting resistance to androgen receptor (AR) targeted therapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to AR targeted therapy in the prostate cancer patient, wherein the biomarker signature comprises presence of AR N-terminal positive CTCs and AR C-terminal loss CTCs. In some embodiments, the resistance is *de novo* resistance.

In further embodiments, the present disclosure provides a method of predicting resistance to androgen receptor (AR) targeted therapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to AR targeted therapy in the prostate cancer patient, wherein the biomarker signature comprises presence of AR N-terminal positive CTCs. In some embodiments, the resistance is *de novo* resistance.

In further embodiments, the present disclosure provides a method of predicting resistance to androgen receptor (AR) targeted therapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to AR targeted therapy in the prostate cancer patient, wherein the biomarker signature comprises increased heterogeneity of the CTCs compared to a reference population. In some embodiments, the resistance is *de novo* resistance.

The present disclosure also provides a method of predicting resistance to chemotherapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to chemotherapy in the prostate cancer patient. In some embodiments, the resistance is de novo resistance. In some embodiments, the resistance is *de novo* resistance.

The present disclosure also provides a method of predicting resistance to chemotherapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to chemotherapy in the prostate cancer patient, wherein the biomarker signature comprises CK+, AR-, nucleoli+, small size in a subpopulation of said CTCs. In some embodiments, the resistance is *de novo* resistance.

The present disclosure also provides a method of predicting resistance to taxane-based chemotherapy in a cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to taxane-based chemotherapy in the prostate cancer patient, wherein the biomarker signature comprises CK+, AR-, nucleoli+, small size in a subpopulation of said CTCs. In some embodiments, the cancer is prostate cancer. In some embodiments, the taxane-based chemotherapy comprises docetaxel or cabazitaxel. In some embodiments, the resistance is de novo resistance. In some embodiments, the resistance is *de novo* resistance.

The present disclosure also provides a method of predicting resistance to taxane-based chemotherapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of resistance to taxane-taxane chemotherapy in the prostate cancer patient, wherein the biomarker signature comprises CK+, AR-, nucleoli+, small size in a subpopulation of said CTCs. In some embodiments, the taxane-based chemotherapy comprises docetaxel or cabazitaxel. In some embodiments, the resistance is *de novo* resistance.

The present disclosure also provides a method of predicting *de novo* resistance to taxane-based chemotherapy in a prostate cancer patient comprising (a) performing a direct analysis comprising immunofluorescent staining and morphological characterization of nucleated cells in a blood sample obtained from the patient to identify circulating tumor cells (CTCs), and (b) based on said direct analysis further determining the presence of a biomarker signature that is predictive of *de novo* resistance to taxane-taxane chemotherapy in the prostate cancer patient, wherein the biomarker signature comprises CK+, AR-, nucleoli+, small size in a subpopulation of said CTCs. In some embodiments, the taxane-based chemotherapy comprises docetaxel or cabazitaxel.

The invention provides a method of predicting resistance to androgen receptor (AR) targeted therapy in a prostate cancer patient comprising
(a) performing a direct analysis comprising immunofluorescent staining and morphological characterization of nucleated cells in a blood sample obtained from the patient to identify circulating tumor cells (CTCs), wherein the direct analysis means the detection of CTCs in the context of all surrounding nucleated cells present in the sample as opposed to after enrichment of the sample for CTCs prior to detection; and
(b) based on said direct analysis further determining the presence of a biomarker signature that is predictive of resistance to AR targeted therapy in the prostate cancer patient,
wherein the biomarker signature comprises a subpopulation of said CTCs which is cytokeratin positive (CK+), AR positive (AR+), and comprises nucleoli (nucleoli+),
and wherein said CTCs are further characterized by AR N-terminal positive staining and AR C-terminal loss.

In certain aspects of the invention, the immunofluorescent staining of nucleated cells comprises pan cytokeratin (CK), cluster of differentiation (CD) 45, diamidino-2-phenylindole (DAPI) and AR. In additional aspects, the AR immunofluorescent staining comprises N- terminal and C-terminal AR nuclear staining.

In some aspects of the invention, the prediction of resistance to chemotherapy or to AR targeted therapy informs a subsequent treatment decision. In particular embodiments, the prostate cancer is metastatic castration resistant prostate cancer (mCRPC).

Androgens in the form of testosterone or the more potent dihydrotestosterone (DHT) have been well-defined drivers of progression of prostate cancer and differentiation of the prostate gland. As such, the backbone of treatment for advanced prostate cancers was established decades ago when castration in the form of surgical orchiectomy achieved significant prostate tumor regression. Since then, substitution to chemical castration has been employed mostly due to patient preference. Androgen Deprivation Therapy (ADT) has therefore become the standard systemic treatment for locally advanced or metastatic prostate cancer. While ADT is almost always effective in most patients, disease progression to castration resistance inevitably occurs. It is now recognized that the androgen receptor (AR) remains overexpressed despite seemingly castrate levels of testosterone, since alternative receptors may activate the AR or other target genes may help perpetuate the castrate-resistant phenotype, hence the term "castration-resistance" has become widely adopted in the literature.

The androgen receptor axis is a validated target for the treatment of castration-resistant prostate cancer. Several perturbations in this pathway are postulated to lead to androgen-independent growth, including androgen receptor mutation and amplification as well as the autocrine production of testosterone. Two drugs targeting this pathway in castration-resistant prostate cancer-abiraterone acetate (Zytiga®) and enzalutamide (Xtandi®)-are approved for use in patients who have already received chemotherapy. Mechanistically, abiraterone exerts antitumor activity by inhibition of the 17,20-lyase pathway, crucial to testosterone synthesis. Enzalutamide binds to the androgen receptor and prevents its translocation into the nucleus. Abiraterone is also approved for patients in the prechemotherapy setting based on results of recent clinical trials.

With new systemic therapies available, the optimal treatment sequence of these drugs in mCRPC becomes increasingly important. Chemotherapy is often indicated in the treatment of castration-resistant prostate cancer, and *taxane-based agents,* such as *docetaxel,* are often the first choice. *Abiraterone (Zytiga),* an anti-androgen drug, was initially only approved for the treatment of castration-resistant prostate cancer after chemotherapy. However, due to successful treatment results many physicians currently prescribe *abiraterone* early during the course of treatment. Combining abiraterone with enzalutamide appears safe, and the efficacy of this combination over enzalutamide alone is being presently being evaluated to detect a survival advantage for the combination approach as upfront treatment as compared to the expected standard sequential use of these agents. Recent studies have suggested that since *taxane-based chemotherapy* and *abiraterone* are effective partially due to similar mechanisms, prior *abiraterone* treatment may contribute to *taxane* resistance and decreased chemotherapy effectiveness, suggesting clinical cross-resistance. The present methods enable optimal sequencing of care between hormone agents (abiraterone and enzalutamide) and cytotoxic chemotherapy (docetaxel and cabazitaxel).

It must be noted that, as used in this specification and the appended claims, the singular forms "a", "an" and "the" include plural referents unless the content clearly dictates otherwise. Thus, for example, reference to "a biomarker" includes a mixture of two or more biomarkers, and the like.

The term "about," particularly in reference to a given quantity, is meant to encompass deviations of plus or minus five percent.

As used in this application, including the appended claims, the singular forms "a," "an," and "the" include plural references, unless the content clearly dictates otherwise, and are used interchangeably with "at least one" and "one or more."

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

A "biomarker" is any molecule, property, characteristic or aspect that can be measured and correlated with the probability for prostate cancer, in particular, mCRPC. The term further encompasses any property, characteristic, feature or aspect of a CTC that can be measured and correlated in connection with predicting resistance to a prostate cancer therapy, for example, AR targeted therapy or taxane based chemotherapy. For a CTC biomarker, such a measurable feature can include, for example, the presence, absence, or concentration of the biomarker, or a subtype thereof, in the biological sample, an altered protein expression, immunofuorescent and/or morphological phenotype, such as, for example, pan cytokeratin (CK), cluster of differentiation (CD) 45, diamidino-2-phenylindole (DAPI) and AR, N-terminal and C-terminal AR nuclear staining, nuclear detail, nuclear contour, presence or absence of nucleoli, quality of cytoplasm, quantity of cytoplasm, intensity of immunofluorescent staining patterns in comparison to the appropriate control subjects, and/or the presence or degree of heterogeneity of phenotypes observed for a CTC biomarker.

In addition to CTC biomarkers, biomarkers can further include risk indicia including, for example age, family history, race and diet. Several factors are associated with increased risk for prostate cancer. Genetics, increasing age, and environmental and geographical factors play a major role. But, dietary factors such as high consumption of fats - fatty acids, alpha linolenic acid found in red meat, etc., deficiency of trace element like selenium and low levels of vitamin D and E have also been implicated in increased risk of development of prostate cancer in some individuals.

As used herein, the term "biomarker signature" refers to a combination comprising two or more biomarkers. The number of biomarkers useful for a biomarker signature is based on the resistance and specificity value for the particular combination of biomarker values.

The term "patient," as used herein preferably refers to a human, but also encompasses other mammals. It is noted that, as used herein, the terms "organism," "individual," "subject," or "patient" are used as synonyms and interchangeably.

The term "androgen receptor targeted therapy" or "AR targeted therapy" in the context of the methods described herein encompasses any therapy that directly or indirectly inhibits the AR signaling pathway, including, for example, through inactivation of androgen production, inactivation of androgen binding to the AR or inactivation of the AR directly. Any therapy that disrupts the AR signaling axis and inhibits the signaling pathway is therefore included in the term AR targeted therapy. An example of direct inhibition, enzalutamide, also known as MDV3100, is one of the most frequently studied AR antagonists. Enzalutamide targets several steps in the AR-signaling pathway. Due to its increased binding affinity for the AR, it is able to block androgens from binding to the receptor, preventing nuclear translocation of the AR, DNA binding, and co-activator recruitment of the ligand-receptor complex. One of the more notable characteristics of enzalutamide is that it is able to bind and inhibit not only wild type, but also mutant AR, which means point mutations of the AR that commonly occur after the progression of PCa which causes castration resistance. Another emerging agent in the treatment of CRPC is abiraterone acetate. While enzalutamide targets the AR directly, abiraterone acts by indirectly inhibiting the AR signaling pathway. CYP17, an enzyme of the cytochrome P450 family, is inhibited by abiraterone. This inhibition is significant because CYP17 plays a critical role in testosterone synthesis. Accordingly, inhibition of causes inhibition of testosterone synthesis, limiting the amount of androgens circulating in the body, thus also limiting the action of the AR. While castration is able to decrease testosterone and DHT synthesis, it does not remove all possible sources of androgens within the body, such as intratumoral or adrenal androgens. As will be understood by those skilled in the art, any mechanism of androgen inhibition is a potential avenue for AR targeted therapy.

As used herein, the term "resistance" in the context of AR targeted therapy or chemotherapy, including taxanes, means that the subject does not show a response to the therapy based on an underlying ability of tumor cells to escape the effect of the therapeutic agent. Resistance includes *de novo* resistance and acquired resistance. Cancer patients that exhibit *de novo* resistance do not respond to chemotherapy from the start. However, in acquired resistance, the cancer cells initially respond to a chemotherapeutic drug but eventually acquire resistance to it. The cells might also show cross-resistance to other structurally and mechanistically unrelated drugs - a phenomenon commonly known as multi drug resistance (MDR). Owing to acquisition of MDR, treatment regimens that combine multiple agents with different targets are no longer effective.

As used herein, the term "CTC heterogeneity" refers to the diversity of CTCs within a sample. Heterogeneity can be determined by categorizing CTCs into discrete types and counting the number of each type we see in the sample. More types of CTCs means that a more diverse population of CTCs were found. For example, for patients X and Y in Table 1, with the following numbers of CTC subtypes, Patient X has greater heterogeneity than Patient Y, even though they have the same number of cells. As described herein, the presence of a greater degree of heterogeneity among CTCs or an increase in heterogeneity compared to a reference level can be indicative of resistance to AR targeted therapy.

**Table 1. CTC Heterogeneity**

| # of subtypes | Patient X | Patient Y |
|---|---|---|
| Type A | 5 | 0 |
| Type B | 5 | 0 |
| Type C | 5 | 15 |

As used herein, the term "circulating tumor cell" or "CTC" is meant to encompass any rare cell that is present in a biological sample and that is related to prostate cancer. CTCs, which can be present as tradiotnal or non-traditinal single cell CTCs or in clusters of CTCs, are often epithelial cells shed from solid tumors found in very low concentrations in the circulation of patients. Included in the term "CTCs" are "traditional CTCs" which are futher defined as a single CTC that is cytokeratin positive, CD45 negative, contains a DAPI nucleus, and is morphologically distinct from surrounding white blood cells. Also encompassed in the term "CTC" are "non-traditional CTCs" which refer to a CTC that differs from a traditional CTC in at least one characteristic. Non-traditional CTCs include CTC clusters, CK negative CTCs that are positive at least one additional biomarker that allows classification as a CTC , small CTCs, nucleoli ⁺ CTCs and CK speckled CTCs. The term "small CTC" refers to a CTC that is the same or smaller in size the the average size of WBCs in the sample. The term "CTC cluster" also is encompassed in the definition of "CTCs" and further means two or more CTCs with touching cell membranes.

In some embodiments, the frequency of AR C-terminal truncated CTCs is a biomarker useful for practicing the methods of the invention. As disclosed herein, decrease in nuclear C-terminal AR staining as compared to N- terminal AR nuclear staining can be part of a biomarker signature predictive of resistance to AR targeted therapy. Furthermore, a decrease in nuclear C-terminal AR staining as compared to N- terminal AR nuclear staining can be part of a biomarker signature predictive of resistance to CYP17 inhibitors such as orteronel, galeterone and VT-464, PP2A activators, and AR N terminal targeting drugs, such as EPI-001, EPI-002 and EPI-506.

In its broadest sense, a biological sample can be any sample that contains CTCs. A sample can comprise a bodily fluid such as blood; the soluble fraction of a cell preparation, or an aliquot of media in which cells were grown; a chromosome, an organelle, or membrane isolated or extracted from a cell; genomic DNA, RNA, or cDNA in solution or bound to a substrate; a cell; a tissue; a tissue print; a fingerprint; cells; skin, and the like. A biological sample obtained from a subject can be any sample that contains cells and encompasses any material in which CTCs can be detected. A sample can be, for example, whole blood, plasma, saliva or other bodily fluid or tissue that contains cells.

In particular embodiments, the biological sample is a blood sample. As described herein, a sample can be whole blood, more preferably peripheral blood or a peripheral blood cell fraction. As will be appreciated by those skilled in the art, a blood sample can include any fraction or component of blood, without limitation, T-cells, monocytes, neutrophiles, erythrocytes, platelets and microvesicles such as exosomes and exosome-like vesicles. In the context of this disclosure, blood cells included in a blood sample encompass any nucleated cells and are not limited to components of whole blood. As such, blood cells include, for example, both white blood cells (WBCs) as well as rare cells, including CTCs.

The samples of this disclosure can each contain a plurality of cell populations and cell subpopulation that are distinguishable by methods well known in the art (*e.g.,* FACS, immunohistochemistry). For example, a blood sample can contain populations of nonnucleated cells, such as erythrocytes (e.g., 4-5 million/µl) or platelets (150,000-400,000 cells/µl), and populations of nucleated cells such as WBCs (*e.g.,* 4,500 - 10,000 cells/µl), CECs or CTCs (circulating tumor cells; *e.g.,* 2-800 cells/). WBCs may contain cellular subpopulations of, *e.g*., neutrophils (2,500-8,000 cells/µl), lymphocytes (1,000-4,000 cells/µl), monocytes (100-700 cells/µl), eosinophils (50-500 cells/µl), basophils (25 - 100 cells/ µl and the like. The samples of this disclosure are non-enriched samples, *i.e.,* they are not enriched for any specific population or subpopulation of nucleated cells. For example, non-enriched blood samples are not enriched for CTCs, WBC, B-cells, T-cells, NK-cells, monocytes, or the like.

In some aspects the sample is a blood sample obtained from a healthy subject or a subject deemed to be at high risk for protate cancer or metastasis of existing prostate cancer based on art known clinically established criteria including, for example, age, race, family snd history. In some embodiments the blood sample is from a subject who has been diagnosed with prostate cancer and/or mCRPC based on tissue or liquid biopsy and/or surgery or clinical grounds. In some embodiments, the blood sample is obtained from a subject showing a clinical manifestation of prostate cancer and/or mCRPC well known in the art or who presents with any of the known risk factors for prostate cancer and/or mCRPC. In particular embodiments, the patient is afflicted with mCRPC.

As used herein in the context of identifying CTCs in a sample, the term "direct analysis" means that the CTCs are detected in the context of all surrounding nucleated cells present in the sample as opposed to after enrichment of the sample for CTCs prior to detection. In some embodiments, the methods comprise microscopy providing a field of view that includes both CTCs and at least 200 surrounding white blood cells (WBCs).

A fundamental aspect of the present disclosure is the unparalleled robustness of the disclosed methods with regard to the detection of CTCs. The rare event detection disclosed herein with regard to CTCs is based on a direct analysis, *i.e.* non-enriched, of a population that encompasses the identification of rare events in the context of the surrounding non-rare events. Identification of the rare events according to the disclosed methods inherently identifies the surrounding events as non-rare events. Taking into account the surrounding non-rare events and determining the averages for non-rare events, for example, average cell size of non-rare events, allows for calibration of the detection method by removing noise. The result is a robustness of the disclosed methods that cannot be achieved with methods that are not based on direct analysis, but that instead compare enriched populations with inherently distorted contextual comparisons of rare events. The robustness of the direct analysis methods disclosed herein enables characterization of CTC, including subtypes of CTCs described herein, that allows for identification of phenotypes and heterogeneity that cannot be achieved with other CTC detection methods and that enables the analysis of biomarkers in the context of the claimed methods.

In some embodiments, the methods for predicting resistance to androgen receptor (AR) targeted therapy or chemotherapy in a tumor of a prostate cancer patient can further take encompass individual patient risk factors and imaging data, which includes any form of imaging modality known and used in the art, for example and without limitation, by X-ray computed tomography (CT), ultrasound, positron emission tomography (PET), electrical impedance tomography and magnetic resonance (MRI). It is understood that one skilled in the art can select an imaging modality based on a variety of art known criteria. As described herein, the methods of the invention can encompass one or more pieces of imaging data. In the methods disclosed herein, one or more individual risk factors can be selected from the group consisting of age, race, family history. It is understood that one skilled in the art can select additional individual risk factors based on a variety of art known criteria. As described herein, the methods of the invention can encompass one or more individual risk factors. Accordingly, biomarkers can include imaging data, individual risk factors and CTC data. As described herein, biomarkers also can include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins) as well as portions or fragments of a biological molecule.

CTCs can be be identified by any suitable method including, for example, by both morphological features and immunofluorescent features. As will be understood by those skilled in the art, biomarkers can include a biological molecule, or a fragment of a biological molecule, the change and/or the detection of which can be correlated, individually or combined with other measurable features, with prostate cancer and/or mCRPC. CTCs, which can be present a single cells or in clusters of CTCs, are often epithelial cells shed from solid tumors and are present in very low concentrations in the circulation of subjects. Accordingly, detection of CTCs in a blood sample can be referred to as rare event detection. CTCs have an abundance of less than 1:1,000 in a blood cell population, *e.g*., an abundance of less than 1:5,000, 1:10,000, 1:30,000, 1:50:000, 1:100,000, 1:300,000, 1:500,000, or 1:1,000,000. In some embodiments, the a CTC has an abundance of 1:50:000 to 1:100,000 in the cell population.

The samples of this disclosure may be obtained by any means, including, *e.g.,* by means of solid tissue biopsy or fluid biopsy (*see, e.g.,* Marrinucci D. et al., 2012, Phys. Biol. 9 016003). Briefly, in particular embodiments, the process can encompass lysis and removal of the red blood cells in a 7.5 mL blood sample, deposition of the remaining nucleated cells on specialized microscope slides, each of which accommodates the equivalent of roughly 0.5 mL of whole blood. A blood sample may be extracted from any source known to include blood cells or components thereof, such as venous, arterial, peripheral, tissue, cord, and the like. The samples may be processed using well known and routine clinical methods (e.g., procedures for drawing and processing whole blood). In some embodiments, a blood sample is drawn into anti-coagulent blood collection tubes (BCT), which may contain EDTA or Streck Cell-Free DNA™. In other embodiments, a blood sample is drawn into CellSave® tubes (Veridex). A blood sample may further be stored for up to 12 hours, 24 hours, 36 hours, 48 hours, or 60 hours before further processing.

In some embodiments, the methods of this disclosure comprise an intitial step of obtaining a white blood cell (WBC) count for the blood sample. In certain embodiments, the WBC count may be obtained by using a HemoCue® WBC device (Hemocue, Ängelholm, Sweden). In some embodiments, the WBC count is used to determine the amount of blood required to plate a consistent loading volume of nucleated cells per slide and to calculate back the equivalent of CTCs per blood volume.

In some embodiments, the methods of this disclosure comprise an initial step of lysing erythrocytes in the blood sample. In some embodiments, the erythrocytes are lysed, *e.g.,* by adding an ammonium chloride solution to the blood sample. In certain embodiments, a blood sample is subjected to centrifugation following erythrocyte lysis and nucleated cells are resuspended, *e.g*., in a PBS solution.

In some embodiments, nucleated cells from a sample, such as a blood sample, are deposited as a monolayer on a planar support. The planar support may be of any material, *e.g.,* any fluorescently clear material, any material conducive to cell attachment, any material conducive to the easy removal of cell debris, any material having a thickness of < 100 µm. In some embodiments, the material is a film. In some embodiments the material is a glass slide. In certain embodiments, the method encompasses an initial step of depositing nucleated cells from the blood sample as a monolayer on a glass slide. The glass slide can be coated to allow maximal retention of live cells (*See, e.g.,* Marrinucci D. et al., 2012, Phys. Biol. 9 016003). In some embodiments, about 0.5 million, 1 million, 1.5 million, 2 million, 2.5 million, 3 million, 3.5 million, 4 million, 4.5 million, or 5 million nucleated cells are deposited onto the glass slide. In some embodiments, the methods of this disclosure comprise depositing about 3 million cells onto a glass slide. In additional embodiments, the methods of this disclosure comprise depositing between about 2 million and about 3 million cells onto the glass slide. In some embodiments, the glass slide and immobilized cellular samples are available for further processing or experimentation after the methods of this disclosure have been completed.

In some embodiments, the methods of this disclosure comprise an initial step of identifying nucleated cells in the non-enriched blood sample. In some embodiments, the nucleated cells are identified with a fluorescent stain. In certain embodiments, the fluorescent stain comprises a nucleic acid specific stain. In certain embodiments, the fluorescent stain is diamidino-2-phenylindole (DAPI). In some embodiments, immunofluorescent staining of nucleated cells comprises pan cytokeratin (CK), cluster of differentiation (CD) 45 and DAPI. In some embodiments further described herein, CTCs comprise distinct immunofluorescent staining from surrounding nucleated cells. In some embodiments, the distinct immunofluorescent staining of CTCs comprises DAPI (+), CK (+) and CD 45 (-). In some embbodiments, the identification of CTCs further comprises comparing the intensity of pan cytokeratin fluorescent staining to surrounding nucleated cells. In some embodiments, the identification of CTC encompasses fluorescent scanning microscopy to detect immunofluorescent staining of nucleated cells in a blood sample. Marrinucci D. et al., 2012, Phys. Biol. 9 016003).

In particular embodiments, all nucleated cells are retained and immunofluorescently stained with monoclonal antibodies targeting cytokeratin (CK), an intermediate filament found exclusively in epithelial cells, a pan leukocyte specific antibody targeting the common leukocyte antigen CD45, and a nuclear stain, DAPI. The nucleated blood cells can be imaged in multiple fluorescent channels to produce high quality and high resolution digital images that retain fine cytologic details of nuclear contour and cytoplasmic distribution. While the surrounding WBCs can be identified with the pan leukocyte specific antibody targeting CD45, CTCs can be identified as DAPI (+), CK (+) and CD 45 (-). In the methods described herein, the CTCs comprise distinct immunofluorescent staining from surrounding nucleated cells.

In further embodiments, CTCs include traditional CTCs also known as high definition CTCs (HD-CTCs) . Traditional CTCs are CK positive, CD45 negative, contain an intact DAPI positive nucleus without identifiable apoptotic changes or a disrupted appearance, and are morphologically distinct from surrounding white blood cells (WBCs). DAPI (+), CK (+) and CD45 (-) intensities can be categorized as measurable features during HD-CTC enumeration as previously described (Figure 1). Nieva et al., Phys Biol 9:016004 (2012). The enrichment-free, direct analysis employed by the methods disclosed herein results in high sensitivity and high specificity, while adding high definition cytomorphology to enable detailed morphologic characterization of a CTC population known to be heterogeneous.

While CTCs can be identified as comprises DAPI (+), CK (+) and CD 45 (-) cells, the methods of the disclosure can be practiced with any other biomarkers such as AR(+) and nucleoli (+) that one of skill in the art selects for characterizing CTCs and/or identifying CTCs and CTC clusters. One skilled in the art knows how to select a morphological feature, biological molecule, or a fragment of a biological molecule, the change and/or the detection of which can be correlated with a CTC. Molecule biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins). The term also encompasses portions or fragments of a biological molecule, for example, peptide fragment of a protein or polypeptide

A person skilled in the art will appreciate that a number of methods can be used to identify CTCs, including microscopy based approaches, including fluorescence scanning microscopy (*see, e.g.,* Marrinucci D. et al., 2012, Phys. Biol. 9 016003), mass spectrometry approaches, such as MS/MS, LC-MS/MS, multiple reaction monitoring (MRM) or SRM and product-ion monitoring (PIM) and also including antibody based methods such as immunofluorescence, immunohistochemistry, immunoassays such as Western blots, enzymelinked immunosorbant assay (ELISA), immunopercipitation, radioimmunoassay, dot blotting, and FACS. Immunoassay techniques and protocols are generally known to those skilled in the art (Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and noncompetitive immunoassays, can be used (Self et al., Curr. Opin. Biotechnol., 7:60-65 (1996), *see also* John R. Crowther, The ELISA Guidebook, 1st ed., Humana Press 2000, ISBN 0896037282 and, An Introduction to Radioimmunoassay and Related Techniques, by Chard T, ed., Elsevier Science 1995, ISBN 0444821198).

A person of skill in the art will futher appreciate that the presence or absence of biomarkers may be detected using any class of marker-specific binding reagents known in the art, including, *e.g*., antibodies, aptamers, fusion proteins, such as fusion proteins including protein receptor or protein ligand components, or biomarker-specific small molecule binders. In some embodiments, the presence or absence of CK or CD45 is determined by an antibody.

The antibodies of this disclosure bind specifically to a biomarker. The antibody can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). The antibody can be any immunoglobulin or derivative thereof, whether natural or wholly or partially synthetically produced. All derivatives thereof which maintain specific binding ability are also included in the term. The antibody has a binding domain that is homologous or largely homologous to an immunoglobulin binding domain and can be derived from natural sources, or partly or wholly synthetically produced. The antibody can be a monoclonal or polyclonal antibody. In some embodiments, an antibody is a single chain antibody. Those of ordinary skill in the art will appreciate that antibody can be provided in any of a variety of forms including, for example, humanized, partially humanized, chimeric, chimeric humanized, *etc.* The antibody can be an antibody fragment including, but not limited to, Fab, Fab', F(ab')2, scFv, Fv, dsFv diabody, and Fd fragments. The antibody can be produced by any means. For example, the antibody can be enzymatically or chemically produced by fragmentation of an intact antibody and/or it can be recombinantly produced from a gene encoding the partial antibody sequence. The antibody can comprise a single chain antibody fragment. Alternatively or additionally, the antibody can comprise multiple chains which are linked together, for example, by disulfide linkages, and any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

A detectable label can be used in the methods described herein for direct or indirect detection of the biomarkers when identifying CTCs in the methods of the invention. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the biomarkers in the methods of the invention. Suitable detectable labels include, but are not limited to, fluorescent dyes (e.g., fluorescein, fluorescein isothiocyanate (FITC), Oregon Green™, rhodamine, Texas red, tetrarhodimine isothiocynate (TRITC), Cy3, Cy5, Alexa Fluor® 647, Alexa Fluor® 555, Alexa Fluor® 488), fluorescent markers (e.g., green fluorescent protein (GFP), phycoerythrin, etc.), enzymes (*e.g*., luciferase, horseradish peroxidase, alkaline phosphatase, etc.), nanoparticles, biotin, digoxigenin, metals, and the like.

For mass-sectrometry based analysis, differential tagging with isotopic reagents, *e.g*., isotope-coded affinity tags (ICAT) or the more recent variation that uses isobaric tagging reagents, iTRAQ (Applied Biosystems, Foster City, Calif.), followed by multidimensional liquid chromatography (LC) and tandem mass spectrometry (MS/MS) analysis can provide a further methodology in practicing the methods of this disclosure.

A chemiluminescence assay using a chemiluminescent antibody can be used for sensitive, non-radioactive detection of proteins. An antibody labeled with fluorochrome also can be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase, beta.-galactosidase are well known in the art.

A signal from the direct or indirect label can be analyzed, for example, using a microscope, such as a fluorescence microscope or a fluorescence scanning microscope. Alternatively, a spectrophotometer can be used to detect color from a chromogenic substrate; a radiation counter to detect radiation such as a gamma counter for detection of ¹²⁵I; or a fluorometer to detect fluorescence in the presence of light of a certain wavelength. If desired, assays used to practice the methods of this disclosure can be automated or performed robotically, and the signal from multiple samples can be detected simultaneously.

In some embodiments, the biomarkers are immunofluorescent markers. In some embodiments, the immunofluorescent makers comprise a marker specific for epithelial cells In some embodiments, the immunofluorescent makers comprise a marker specific for white blood cells (WBCs). In some embodiments, one or more of the immunofluorescent markers comprise CD 45 and CK.

In some embodiments, the presence or absence of immunofluorescent markers in nucleated cells, such as CTCs or WBCs, results in distinct immunofluorescent staining patterns. Immunofluorescent staining patterns for CTCs and WBCs may differ based on which epithelial or WBC markers are detected in the respective cells. In some embodiments, determining presence or absence of one or more immunofluorescent markers comprises comparing the distinct immunofluorescent staining of CTCs with the distinct immunofluorescent staining of WBCs using, for example, immunofluorescent staining of CD45, which distinctly identifies WBCs. There are other detectable markers or combinations of detectable markers that bind to the various subpopulations of WBCs. These may be used in various combinations, including in combination with or as an alternative to immunofluorescent staining of CD45.

In some embodiments, CTCs comprise distinct morphological characteristics compared to surrounding nucleated cells. In some embodiments, the morphological characteristics comprise nucleus size, nucleus shape, cell size, cell shape, and/or nuclear to cytoplasmic ratio. In some embodiments, the method further comprises analyzing the nucleated cells by nuclear detail, nuclear contour, presence or absence of nucleoli, quality of cytoplasm, quantity of cytoplasm, intensity of immunofluorescent staining patterns. A person of ordinary skill in the art understands that the morphological characteristics of this disclosure may include any feature, property, characteristic, or aspect of a cell that can be determined and correlated with the detection of a CTC.

CTCs can be characterized with any microscopic method known in the art. In some embodiments, the method is performed by fluorescent scanning microscopy. In certain embodiments the microscopic method provides high-resolution images of CTCs and their surrounding WBCs (*see, e.g.,* Marrinucci D. et al., 2012, Phys. Biol. 9 016003)). In some embodiments, a slide coated with a monolayer of nucleated cells from a sample, such as a non-enriched blood sample, is scanned by a fluorescent scanning microscope and the fluorescence intensities from immunofluorescent markers and nuclear stains are recorded to allow for the determination of the presence or absence of each immunofluorescent marker and the assessment of the morphology of the nucleated cells. In some embodiments, microscopic data collection and analysis is conducted in an automated manner.

In some embodiments, identification of CTCs via direct analysis includes detecting one or more biomarkers, for example, CK and CD 45. A biomarker is considered "present" in a cell if it is detectable above the background noise of the respective detection method used (*e.g.,* 2-fold, 3-fold, 5-fold, or 10-fold higher than the background; *e.g.,* 2σ or 3σ over background). In some embodiments, a biomarker is considered "absent" if it is not detectable above the background noise of the detection method used *(e.g.,* <1.5-fold or <2.0-fold higher than the background signal; *e.g.,* <1.5σ or <2.0σ over background).

In some embodiments, the presence or absence of immunofluorescent markers in nucleated cells is determined by selecting the exposure times during the fluorescence scanning process such that all immunofluorescent markers achieve a pre-set level of fluorescence on the WBCs in the field of view. Under these conditions, CTC-specific immunofluorescent markers, even though absent on WBCs are visible in the WBCs as background signals with fixed heights. Moreover, WBC-specific immunofluorescent markers that are absent on CTCs are visible in the CTCs as background signals with fixed heights. A cell is considered positive for an immunofluorescent marker (*i.e.,* the marker is considered present) if its fluorescent signal for the respective marker is significantly higher than the fixed background signal *(e.g.,* 2-fold, 3-fold, 5-fold, or 10-fold higher than the background; e.g., 2σ or 3σ over background). For example, a nucleated cell is considered CD 45 positive (CD 45⁺) if its fluorescent signal for CD 45 is significantly higher than the background signal. A cell is considered negative for an immunofluorescent marker (*i.e.,* the marker is considered absent) if the cell's fluorescence signal for the respective marker is not significantly above the background signal *(e.g.,* <1.5-fold or <2.0-fold higher than the background signal; *e.g.,* <1.5σ or <2.0σ over background).

Typically, each microscopic field contains both CTCs and WBCs. In certain embodiments, the microscopic field shows at least 1, 5, 10, 20, 50, or 100 CTCs. In certain embodiments, the microscopic field shows at least 10, 25, 50, 100, 250, 500, or 1,000 fold more WBCs than CTCs. In certain embodiments, the microscopic field comprises one or more CTCs or CTC clusters surrounded by at least 10, 50, 100, 150, 200, 250, 500, 1,000 or more WBCs.

In some embodiments of the methods described herein, identification of CTCs via direct analysis comprises enumeration of CTCs that are present in the blood sample. In some embodiments, the methods described herein encompass detection of at least 1.0 CTC/mL of blood, 1.5 CTCs/mL of blood, 2.0 CTCs/mL of blood, 2.5 CTCs/mL of blood, 3.0 CTCs/mL of blood, 3.5 CTCs/mL of blood, 4.0 CTCs/mL of blood, 4.5 CTCs/mL of blood, 5.0 CTCs/mL of blood, 5.5 CTCs/mL of blood, 6.0 CTCs/mL of blood, 6.5 CTCs/mL of blood, 7.0 CTCs/mL of blood, 7.5 CTCs/mL of blood, 8.0 CTCs/mL of blood, 8.5 CTCs/mL of blood, 9.0 CTCs/mL of blood, 9.5 CTCs/mL of blood, 10 CTCs/mL of blood, or more.

In some embodiments of methods described herein, identification of CTCs comprises detecting distinct subtypes of CTCs, including non-traditional CTCs. In some embodiments, the methods described herein encompass detection of at least 0.1 CTC cluster/mL of blood, 0.2 CTC clusters/mL of blood, 0.3 CTC clusters/mL of blood, 0.4 CTC clusters/mL of blood, 0.5 CTC clusters/mL of blood, 0.6 CTC clusters/mL of blood, 0.7 CTC clusters/mL of blood, 0.8 CTC clusters/mL of blood, 0.9 CTC clusters/mL of blood, 1 CTC cluster/mL of blood, 2 CTC clusters/mL of blood, 3 CTC clusters/mL of blood, 4 CTC clusters/mL of blood, 5 CTC clusters/mL of blood, 6 CTC clusters/mL of blood, 7 CTC clusters/mL of blood, 8 CTC clusters/mL of blood, 9 CTC clusters/mL of blood, 10 clusters/mL or more. In a particular embodiment, the methods described herein encompass detection of at least 1 CTC cluster/mL of blood.

In some embodiments, the methods predicting resistance to androgen receptor (AR) targeted therapy comprises genomic analysis of the CTCs, for example, by fluorescence in situ hybridization (FISH). In some embodiments, the methods predicting resistance to androgen receptor (AR) targeted therapy comprises detection of erythroblast transformationspecific (ETS)-related gene (*ERG*) rearrangement. In some embodiments, , the methods predicting resistance to androgen receptor (AR) targeted therapy comprises detection of loss of Phosphatase and tensin homolog gene (*PTEN*)*.*

Standard molecular biology techniques known in the art and not specifically described are generally followed as in Sambrook et al., Molecular Cloning: A Laboratory Manual, Cold Spring Harbor Laboratory Press, New York (1989), and as in Ausubel et al., Current Protocols in Molecular Biology, John Wiley and Sons, Baltimore, Md. (1989) and as in Perbal, A Practical Guide to Molecular Cloning, John Wiley & Sons, New York (1988), and as in Watson et al., Recombinant DNA, Scientific American Books, New York and in Birren et al (eds) Genome Analysis: A Laboratory Manual Series, Vols. 1-4 Cold Spring Harbor Laboratory Press, New York (1998). Polymerase chain reaction (PCR) can be carried out generally as in PCR Protocols: A Guide to Methods and Applications, Academic Press, San Diego, Calif. (1990). Any method capable of determining a DNA copy number profile of a particular sample can be used for molecular profiling according to the disclosure provided the resolution is sufficient to identify the biomarkers. The skilled artisan is aware of and capable of using a number of different platforms for assessing whole genome copy number changes at a resolution sufficient to identify the copy number of the one or more biomarkers of the disclosure.

*In situ* hybridization assays are well known and are generally described in Angerer et al., Methods Enzymol. 152:649-660 (1987). In an in situ hybridization assay, cells, e.g., from a biopsy, are fixed to a solid support, typically a glass slide. If DNA is to be probed, the cells are denatured with heat or alkali. The cells are then contacted with a hybridization solution at a moderate temperature to permit annealing of specific probes that are labeled. The probes are preferably labeled with radioisotopes or fluorescent reporters. FISH (fluorescence in situ hybridization) uses fluorescent probes that bind to only those parts of a sequence with which they show a high degree of sequence similarity.

FISH is a cytogenetic technique used to detect and localize specific polynucleotide sequences in cells. For example, FISH can be used to detect DNA sequences on chromosomes. FISH can also be used to detect and localize specific RNAs, e.g., mRNAs, within tissue samples. In FISH uses fluorescent probes that bind to specific nucleotide sequences to which they show a high degree of sequence similarity. Fluorescence microscopy can be used to find out whether and where the fluorescent probes are bound. In addition to detecting specific nucleotide sequences, e.g., translocations, fusion, breaks, duplications and other chromosomal abnormalities, FISH can help define the spatial-temporal patterns of specific gene copy number and/or gene expression within cells and tissues.

In some embodiments, the disclosed methods for predicting resistance to androgen receptor (AR) targeted therapy or chemotherapy in a tumor of a prostate cancer patient encompass the use of a predictive model. In further embodiments, the disclosed methods for predicting resistance to androgen receptor (AR) targeted therapy or chemotherapy in a tumor of a prostate cancer patient encompass comparing a measurable feature with a reference feature. As those skilled in the art can appreciate, such comparison can be a direct comparison to the reference feature or an indirect comparison where the reference feature has been incorporated into the predictive model. In further embodiments, analyzing a measurable feature to prospectively identify resistance to androgen receptor (AR) targeted therapy or chemotherapy in a tumor of a prostate cancer patient encompasses one or more of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, or a combination thereof. In particular embodiments, the analysis comprises logistic regression. In additional embodiments, methods for predicting resistance to androgen receptor (AR) targeted therapy or chemotherapy in a tumor of a prostate cancer patient is expressed as a risk score.

An analytic classification process can use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms and other methods known to those skilled in the art.

Classification can be made according to predictive modeling methods that set a threshold for determining the probability that a sample belongs to a given class. The probability preferably is at least 50%, or at least 60%, or at least 70%, or at least 80%, or at least 90% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model can be evaluated according to its ability to provide a quality metric, e.g. AUROC (area under the ROC curve) or accuracy, of a particular value, or range of values. Area under the curve measures are useful for comparing the accuracy of a classifier across the complete data range. Classifiers with a greater AUC have a greater capacity to classify unknowns correctly between two groups of interest. ROC analysis can be used to select the optimal threshold under a variety of clinical circumstances, balancing the inherent tradeoffs that exist between specificity and sensitivity. In some embodiments, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC of at least about 0.7, at least about 0.75, at least about 0.8, at least about 0.85, at least about 0.9, or higher.

As is known in the art, the relative sensitivity and specificity of a predictive model can be adjusted to favor either the specificity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about 0.7, at least about 0.75, at least about 0.8, at least about 0.85, at least about 0.9, or higher.

The raw data can be initially analyzed by measuring the values for each measurable feature or biomarker, usually in triplicate or in multiple triplicates. The data can be manipulated, for example, raw data can be transformed using standard curves, and the average of triplicate measurements used to calculate the average and standard deviation for each patient. These values can be transformed before being used in the models, *e.g.* logtransformed, Box-Cox transformed (Box and Cox, Royal Stat. Soc., Series B, 26:211-246(1964). The data are then input into a predictive model, which will classify the sample according to the state. The resulting information can be communicated to a patient or health care provider.

In some embodiments, the method disclosed herein for predicting resistance to androgen receptor (AR) targeted therapy or chemotherapy in a tumor of a prostate cancer patient has a specificity of >60%, >70%, >80%, >90% or higher. In additional embodiments, the methods for predicting resistance to androgen receptor (AR) targeted therapy or chemotherapy in a tumor of a prostate cancer patient has a specificity >90% at a classification threshold of 7.5 CTCs/mL of blood.

As will be understood by those skilled in the art, an analytic classification process can use any one of a variety of statistical analytic methods to manipulate the quantitative data and provide for classification of the sample. Examples of useful methods include, without limitation, linear discriminant analysis, recursive feature elimination, a prediction analysis of microarray, a logistic regression, a CART algorithm, a FlexTree algorithm, a LART algorithm, a random forest algorithm, a MART algorithm, and machine learning algorithms.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

The following examples are provided by way of illustration, not limitation.

### EXAMPLES

### Example 1 (not part of the invention). Characterization of CTCs and CTC subpopulations in progressive mCRPC

48 samples from 21 unique progressive mCRPC patients treated with androgen receptor targeted (AR tx) therapies, 9 (43%) on Abiraterone plus Prednisone (AA+P) and 12 (57%) on Enzalutamide (E). Samples were collected and shipped to Epic Sciences, where cells were stained and CTC identified by fluorescent scanners and algorithmic analysis. CTCs, defined as classic (CK+ CD45- w/intact DAPI nuclei and distinct), apopotic (CK+, CD45-, non-intact nuclei) and CK- (CK-, CD45-, intact and distinct) were identified. CTCs reported per mL of blood and were examined for AR, *PTEN* & *ERG.* CTC data were analyzed in context of PSA, Veridex CTC (reported per 7.5mL of blood), and clinical history.

With Epic, all 21 pts had detectable CTCs (mdn 23 cells/ml, range 2 to 249), whereas with Veridex, 14 (67%) pts had ≥ 5 CTC/7.5 ml (mdn 5 cells/7.5 ml, range 0 to ≥200).

**Table 2. Baseline AR Expression (Epic CTC)**

| **AR Targeted Tx; N=21** | **Baseline AR Expression (Epic CTC)** | |
|---|---|---|
| | Low AR expression; N=14 | High AR expression ; N=7 |
| **AA+P;** N=9 | 7 | 2 |
| **E;** N=12 | 7 | 5 |

0/7 (0%) with high AR responded to Abiraterone plus Prednisone (AA+P) or Enzalutamide (E), 8/14 (53%) with low AR had a PSA decline and stable radiographic disease (median follow-up 12 wks). Variations in AR protein expression and localization heterogeneity was seen in all pts to varying degrees. 6/21 (29%) Pts demonstrated CK-CTC/CTC ratio >50% (mdn 9 cells/ml, range 5-38). *ERG* rearrangement & *PTEN loss* were correlated.

Epic CTC analysis provides higher detection rates, while enabling individual cell analyses for predictive biomarkers of resistance to AR directed therapies. Notable was the marked heterogeneity of detection of AR, *ERG,* and *PTEN* of individual CTCs. Studies are ongoing to further explore associations with outcomes.

### Example 2 (not part of the invention). Characterization of CTCs and CTC subpopulations in progressive mCRPC

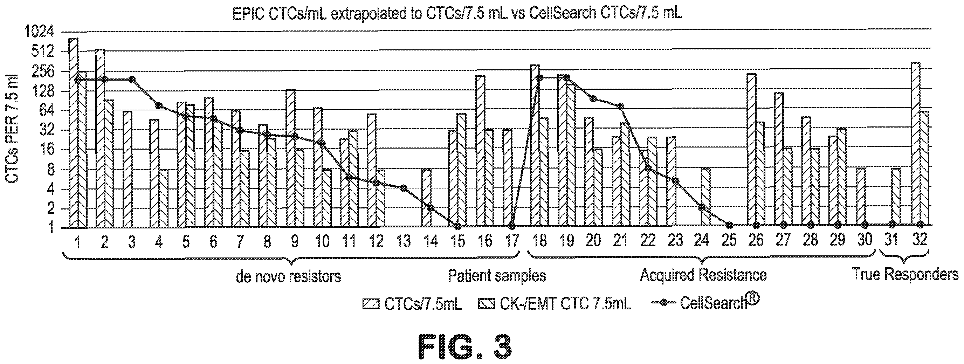

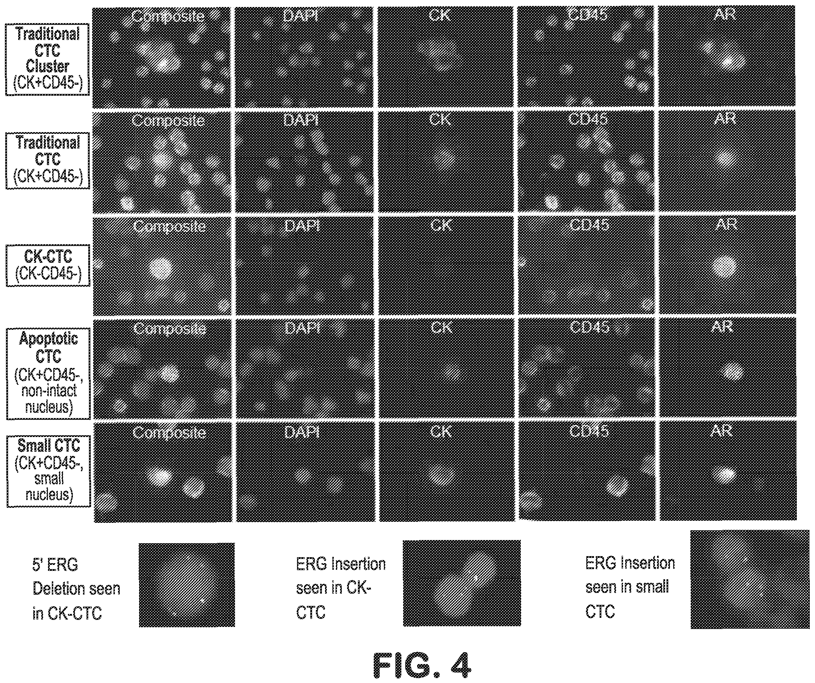

32 samples from 30 unique progressive mCRPC Pts treated with androgen receptor targeted (AR tx) therapies; 14/30 (46.7%) on Abiraterone plus Prednisone (AA+P) and 16/30 (53.3%) on Enzalutamide (E). Samples were collected and shipped to Epic Sciences where cells were stained and CTC identified by fluorescent scanners and algorithmic analysis (Figure 3). CTCs, defined as traditional (CK+CD45- with intact DAPI nuclei and morphologically distinct), apopotic (CK+CD45-, non-intact nuclei) and CK- (CK-CD45-, intact and morphologically distinct) were identified (Figure 4). CTCs reported per mL of blood were examined for AR expression by immunofluorescence (IF), and for PTEN loss and ERG rearrangements by FISH. CTC data were analyzed in context of PSA, CellSearch® CTC count (reported per 7.5mL of blood), and clinical history.

Using Epic Sciences CTC platform, 26/30 (86.7%) pts had > 5 traditional CTCs/7.5 mL of blood (mdn 56 cells/mL, range 8 to >200), whereas with CellSearch®, 15/30 (50.0%) pts had > 5 CTC/7.5 mL (mdn 62 cells/7.5 mL, range 6 to >200) (Figure 3). As shown in Table 3, ean expression of AR >3.82 or >5 CK- CTCs/mL predicted *de novo* resistance vs. acquired resistance (Sensitivity= 63%, Specificity = 83%, p= 0.0235). No true responders had AR>3.82 nor >5 CK- CTCs/mL. ERG rearrangement & PTEN loss also were enriched in de novo resistant population. Neither CellSearch® CTC nor PSA were predictive of *de novo* resistance.

**Table 3. Model for prediction of de novo resistance**

| **Model for prediction of de novo resistance: mean AR expression > 3.82 or CK-CTC** > **38 per 7.5mL** | |
|---|---|
| Sensitivity | 63% |
| Specificity | 86% |
| p value | 0.0106 |

Epic CTC analysis provides higher detection rates, while enabling individual cell analyses for predictive biomarkers of sensitivity to AR directed therapies. Notable was the marked heterogeneity of detection of AR, ERG, and PTEN of individual CTCs. High mean AR expression in all CTC subpopulations and high frequency of CK- CTCs are associated with de novo resistance.

### Example 3. Predictive Biomarkers of Sensitivity to Androgen Receptor Signaling (ARS) and Taxane Based Chemotherapy in Circulating Tumor Cells (CTCs) of Patients (pts) with metastatic Castration Resistant Prostate Cancer (mCRPC)

91 patient blood samples collected from 79 patients for CTC analysis with the Epic Sciences platform prior to treatment (27 pre-A, 28 pre-E, 28 pre-D, 8 pre-C). Epic analysis identified traditional CTCs (CK+, CD45-, intact nuclei, morph distinct), CK- CTCs (CK-, CD45, intact nuclei, morphology distinct), small CTCs (CK+, CD45-, intact nuclei, small cell size), and CTC clusters. If staining for AR N, AR C exp was performed, digital pathology algorithms analyzed CTC morphology. A classifier was developed to associate clinical phenotypes with outcome to a specific agent.

A & E biomarker signatures included: AR N/C exp. & presence of CK+, AR+, Nucleoli+ CTCs. D & C biomarker signatures included: presence of CK+, small, AR-, Nucleoli+ CTCs. Multivariate algorithms for A & E and D & C were statistically associated with *de novo* resistance. Line of therapy was not a univariate predictor of response.

**Table 4. Baseline CTC Signatures**

| **Baseline CTC Signatures** | | | | | |
|---|---|---|---|---|---|
| | Biomarker | % of Pts with Max PSA Decline >50% | Median weeks on study (low-high) | OR | P value |
| A&E (n=55) | + n=22 | 5% | 9 (5-51) | 15 | .0001 |
| | - n=33 | 48% | 29 (5-67) | | |
| C&D (n=36) | + n=12 | 17% | 10 (3-22) | 13 | .01 |
| | - n=24 | 38% | 22 (3-69) | | |

Characterization of CTCs identified predictive biomarkers of sensitivity to ARS Tx & taxane chemotherapy in mCRPC pts. The proposed A&E signature differs from C & D, providing the opportunity to better guide treatment selection and improve patient outcomes using a real-time, non-invasive blood biopsy. Prospective trials to validate results are planned.

## Claims

1. A method of predicting resistance to androgen receptor (AR) targeted therapy in a prostate cancer patient comprising
(a) performing a direct analysis comprising immunofluorescent staining and morphological characterization of nucleated cells in a blood sample obtained from the patient to identify circulating tumor cells (CTCs), wherein the direct analysis means the detection of CTCs in the context of all surrounding nucleated cells present in the sample as opposed to after enrichment of the sample for CTCs prior to detection; and
(b) based on said direct analysis further determining the presence of a biomarker signature that is predictive of resistance to AR targeted therapy in the prostate cancer patient,
wherein the biomarker signature comprises a subpopulation of said CTCs which is cytokeratin positive (CK+), AR positive (AR+), and comprises nucleoli (nucleoli+), and wherein said CTCs are further **characterized by** AR N-terminal positive staining and AR C-terminal loss.

2. The method of claim 1, wherein the immunofluorescent staining of nucleated cells comprises pan cytokeratin, cluster of differentiation 45 (CD45), diamidino-2-phenylindole (DAPI) and AR.

3. The method of claim 1 or 2, wherein the AR immunofluorescent staining comprises N- terminal or C-terminal AR nuclear staining.

4. The method of claim 3, wherein the C-terminal AR nuclear staining is decreased compared to the N-terminal AR nuclear staining.

5. The method of claim 1, wherein said CTCs comprise traditional CTCs, CTC clusters, CK negative (CK⁻) CTCs, and small CTCs,
wherein the traditional CTCs are **characterized by** DAPI+CK+CD45- and are morphologically distinct from surrounding white blood cells (WBCs), and
wherein the small CTCs are **characterized by** DAPI+CK+CD45- and are of the same or smaller size than the average size of WBCs in the sample.

6. The method of claim 1, wherein said prediction informs a subsequent treatment decision.

7. The method of claim 1, wherein the method comprises an initial step of depositing the nucleated cells as a monolayer onto a slide.

8. The method of claim 1, wherein the prostate cancer is metastatic castration resistant prostate cancer (mCRPC).

9. The method of claim 1, wherein the identification of CTCs comprises fluorescent scanning microscopy.

10. The method of claim 9, wherein the fluorescent scanning microscopy provides a field of view comprising both CTCs and at least 200 surrounding white blood cells (WBCs).

11. The method of claim 1, wherein the direct analysis comprises assessing at least 4 million of the nucleated cells.

## Patentansprüche

1. Verfahren zum Prognostizieren der Resistenz gegenüber einer auf den Androgenrezeptor (AR) gerichteten Therapie in einem Prostatakrebspatienten, umfassend
(a) das Durchführen einer direkten Analyse, die eine Immunfluoreszenzfärbung und eine morphologische Charakterisierung von nukleierten Zellen in einer vom Patienten erhaltenen Blutprobe umfasst, um zirkulierende Tumorzellen (CTCs) zu identifizieren, wobei die direkte Analyse, im Gegensatz zum Nachweis nach Anreicherung der Probe für CTCs vor dem Nachweis, den Nachweis von CTCs im Kontext aller umgebenden nukleierten Zellen in der Probe bedeutet; und
(b) basierend auf der direkten Analyse, ferner die Bestimmung des Vorhandenseins einer Biomarkersignatur, die eine Resistenz gegenüber einer auf den AR-gerichteten Therapie in dem Prostatakrebspatienten vorhersagt,
wobei die Biomarkersignatur eine Subpopulation der CTCs umfasst, die Cytokeratinpositiv (CK+) ist, AR-positiv (AR+) ist und Nucleoli (Nucleoli+) umfasst, und wobei die CTCs ferner durch N-terminale positive Färbung des AR und C-terminalen Verlust des AR gekennzeichnet sind.

2. Verfahren nach Anspruch 1, wobei die Immunfluoreszenzfärbung von nukleierten Zellen Pan-Cytokeratin, Differenzierungscluster 45 (CD45), Diamidin-2-phenylindol (DAPI) und AR umfasst.

3. Verfahren nach Anspruch 1 oder 2, wobei die AR-Immunfluoreszenzfärbung eine N-terminale oder C-terminale AR-Kernfärbung umfasst.

4. Verfahren nach Anspruch 3, wobei die C-terminale AR-Kernfärbung im Vergleich zur N-terminalen AR-Kernfärbung vermindert ist.

5. Verfahren nach Anspruch 1, wobei die CTCs herkömmliche CTCs, CTC-Cluster, CKnegative (CK⁻) CTCs und kleine CTCs umfassen, wobei die herkömmlichen CTCs durch DAPI+CK+CD45- gekennzeichnet sind und sich morphologisch von den umgebenden weißen Blutkörperchen (WBK) unterscheiden, und wobei die kleinen CTCs durch DAPI+CK+CD45- gekennzeichnet sind und von gleicher oder geringerer Größe als die durchschnittliche Größe der WBK in der Probe sind.

6. Verfahren nach Anspruch 1, wobei die Prognose Information für die Entscheidung über eine nachfolgende Behandlung liefert.

7. Verfahren nach Anspruch 1, wobei das Verfahren einen ersten Schritt der Ablagerung der nukleierten Zellen als Monoschicht auf einem Objektträger umfasst.

8. Verfahren nach Anspruch 1, wobei der Prostatakrebs ein metastatischer kastrationsresistenter Prostatakrebs (mCRPC) ist.

9. Verfahren nach Anspruch 1, wobei die Identifizierung von CTCs Fluoreszenz-Rastermikroskopie umfasst.

10. Verfahren nach Anspruch 9, wobei die Fluoreszenz-Rastermikroskopie ein Sichtfeld bereitstellt, das sowohl CTCs als auch mindestens 200 umgebende weiße Blutkörperchen (WBK) umfasst.

11. Verfahren nach Anspruch 1, wobei die direkte Analyse ein Bewerten von mindestens 4 Millionen der nukleierten Zellen umfasst.

## Revendications

1. Procédé de prédiction de la résistance à une thérapie ciblant le récepteur des androgènes (AR) chez un patient souffrant d'un cancer de la prostate comprenant
(a) la mise en œuvre d'une analyse directe comprenant une coloration par immunofluorescence et une caractérisation morphologique de cellules nucléées dans un échantillon de sang obtenu à partir du patient pour identifier des cellules tumorales circulantes (CTCs), dans lequel l'analyse directe signifie la détection de CTCs dans le contexte de toutes les cellules nucléées environnantes présentes dans l'échantillon plutôt qu'après l'enrichissement de l'échantillon en CTC avant la détection ; et
(b) sur la base de ladite analyse directe, la détermination ensuite de la présence d'une signature de biomarqueur qui est prédictive d'une résistance à une thérapie ciblant l'AR chez le patient souffrant d'un cancer de la prostate,
dans lequel la signature de biomarqueur comprend une sous-population desdites CTCs, qui est positive à la cytokératine (CK+), positive à l'AR (AR+), et comprend des nucléoles (nucléoles+), et dans lequel lesdites CTCs sont **caractérisées en outre par** une coloration positive à l'extrémité N-terminale de l'AR et une perte de l'extrémité C-terminale de l'AR

2. Procédé selon la revendication 1, dans lequel la coloration par immunofluorescence des cellules nucléées comprend la pan-cytokératine, le cluster de différenciation 45 (CD45), le diamidino-2-phénylindole (DAPI) et l'AR.

3. Procédé selon la revendication 1 ou 2, dans lequel la coloration par immunofluorescence de l'AR comprend une coloration nucléaire N-terminale ou C-terminale de l'AR

4. Procédé selon la revendication 3, dans lequel la coloration nucléaire C-terminale de l'AR est diminuée par rapport à la coloration nucléaire N-terminale de l'AR

5. Procédé selon la revendication 1, dans lequel lesdites CTCs comprennent des CTCs traditionnelles, des agrégats de CTCs, des CTCs négatives à CK (CK⁻), et des petites CTCs, dans lequel les CTCs traditionnelles sont **caractérisées par** DAPI+CK+CD45-et sont morphologiquement distinctes par rapport aux globules blancs environnants, et dans lequel les petites CTCs sont **caractérisées par** DAPI+CK+CD45- et ont une taille inférieure ou égale à la taille moyenne des globules blancs dans l'échantillon.

6. Procédé selon la revendication 1, dans lequel ladite prédiction éclaire une décision de traitement ultérieur.

7. Procédé selon la revendication 1, dans lequel le procédé comprend une étape initiale de dépôt des cellules nucléées sous la forme d'une monocouche sur une lame.

8. Procédé selon la revendication 1, dans lequel le cancer de la prostate est un cancer de la prostate résistant à la castration métastatique (CRPCm).

9. Procédé selon la revendication 1, dans lequel l'identification des CTCs comprend une microscopie à balayage de fluorescence.

10. Procédé selon la revendication 9, dans lequel la microscopie à balayage de fluorescence fournit un champ de vision comprenant à la fois des CTCs et au moins 200 globules blancs environnants.

11. Procédé selon la revendication 1, dans lequel l'analyse directe comprend l'évaluation d'au moins 4 millions des cellules nucléées.
